# EUROPEAN PATENT SPECIFICATION

(11) **EP 1 812 459 B1**
(45) Date of publication and mention of the grant of the patent: **30.03.2011**
(21) Application number: 04793596.0
(22) Date of filing: 28.10.2004
(51) Int. Cl.: C07K 14/47, C07K 1/16, C12N 15/63, C12N 5/10, G01N 33/68

(54) **METHOD FOR THE MASS PRODUCTION OF MULTIMERIC MANNOSE BINDING LECTIN**
VERFAHREN ZUR MASSENHERSTELLUNG VON MULTIMEREM MANNOSEBINDENDEM LECTIN
PROCEDE POUR PRODUIRE EN MASSE DE LA LECTINE DE LIAISON DU MANNOSE MULTIMERE

(43) Date of publication of application: 01.08.2007
(73) Proprietor: Dobeel Co., Ltd., Seongnam-si, Kyonggi-do 462-716 (KR)
(72) Inventor: MOON, Hong, Mo, Kyonggi-do 464-120 (KR); YUM, Jung, Sun, Kyonggi-do 463-914 (KR); AHN, Byung, Cheol, Yonging-si, Gyeonggi-do 448-542 (KR); LEE, Joo, Youn, Kyonggi-do 463-862 (KR); YOON, Jaeseung, Kyonggi-do 449-160 (KR)
(74) Representative: Office Freylinger
(86) International application number: PCT/KR2004/002739
(87) International publication number: WO 2006/046786

(56) References cited:
- WO-A-02/14525
- WO-A-03/010188
- WO-A1-03/018617
- KR-A- 2004 106 194
- US-B1- 6 337 193
- US-B1- 6 589 534
- HOOKER BRADLEY A ET AL: "EXPRESSION AND CHARACTERIZATION OF HUMAN RECOMBINANT MANNOSE BINDING LECTIN AND ITS EFFECTS ON HUMAN IMMUNODEFICIENCY VIRUS ESCAPE MUTANTS" FASEB JOURNAL, FED. OF AMERICAN SOC. FOR EXPERIMENTAL BIOLOGY, BETHESDA, MD, vol. 15, no. 5, 8 March 2001 (2001-03-08), page A1012, XP009077508 ISSN: 0892-6638
- VORUP-JENSEN THOMAS ET AL: "Recombinant expression of human mannan-binding lectin" INTERNATIONAL IMMUNOPHARMACOLOGY, ELSEVIER, AMSTERDAM, NL, vol. 1, no. 4, 1 April 2001 (2001-04-01), pages 677-687, XP002218698 ISSN: 1567-5769
- OHTANI K ET AL: "High-level and effective production of human mannan-binding lectin (MBL) in Chinese hamster ovary (CHO) cells" JOURNAL OF IMMUNOLOGICAL METHODS, ELSEVIER SCIENCE PUBLISHERS B.V.,AMSTERDAM, NL, vol. 222, no. 1-2, 1 January 1999 (1999-01-01), pages 135-144, XP004152435 ISSN: 0022-1759
- LARSEN F. ET AL: 'Disease-associated mutations in human mannose binding lectin compromise oligomerization and activity of the final protein' JOURNAL OF BIOLOGICAL CHEMISTRY vol. 279, no. 20, 2004, pages 21302 - 21311, XP008086641

<110> Dobeel Co., Ltd.
<120> METHOD FOR THE MASS PRODUCTION OF MULTIMERIC MANNOSE BINDING LECTIN
<130> 4FPO-09-02
<140> PCT/KR2004/002739
   <141> 2004-10-28
<160> 2
<170> KopatentIn 1.71
<210> 1
   <211> 34
   <212> DNA
   <213> Artificial Sequence
<220>
   <223> Forward primer 1 for MBL cDNA amplification
<400> 1.
   ctagctagcc accatgtccc tgttt ccatc actc 34
<210> 2
   <211> 29
   <212> DNA
   <213> Artificial Sequence
<220>
   <223> Reverse primer 2 for MBL cDNA amplification
<400> 2
   gaagatctca gat agggaac tcacagacg 29

## Description

### Technical Field

The present invention relates to the development of processes by which the masts of multimeric recombinant human mannose binding lectin (rhMBL) possible.

### Background of the Invention

Mannose binding lectin (referred to as "MBL" hereinafter) is a serum protein involved in natural immunity (innate immunity). The molecular weight of MBL is 32 kDa. MBL is composed of C-terminal carbohydrate recognition domain (CRD), collagen domain, and cystein rich region in the amino terminal end. Three MBL molecules form a complex via triple helix formation using collagen domains, and then up to 6 complexes can combine together by inter-molecular disulfide bond (-*s-s*-) among cystein residues on the N-terminal end, resulting in the formation of multimeric MBL molecule consisting of up to 18 MBL molecules.

Oligomeric form of MBL can form a complex with other proteins such as MBL associated serine proteases (MBSP-1, MASP-2 or MASP-3) or MBL associated protein (Map 19). Although the general molecular structure and the function of MBL involved in complement activation are similar to C1q, which is the first component of complement system, unlike C1q MBL activates complement system by cleaving C4 and C2. This complement activation process called lectin pathway is accomplished through MBL associated serine protease activated upon MBL binding to microbe. Activation of complement system by MBL is one of MBL functions in the primary defense against microbial infection before taking place adaptive immune responses. MBL binding to microorganism through carbohydrate-recognizing domains (CRD) that recognize unique glycosylation patterns of surface protein of a microorganism is the prerequisite of complement activation. MBL binding to microorganism activates the MBL associated precursors of serine proteases, MASP-1 and MASP-2, leading to the cleavage of C4 and C2 of the complement system, generating C4b2a and C3b. Some of the proteins generated in the complement activation directly attach to the surface of a microorganism as opsonin, and MBL can also function as opsonin to make the phagocytosis of MBL bound microbes more efficient by phagocytic cells. Some activated complement proteins directly lyses invading microorganisms by forming membrane attacking complex (MAC), and other complement protein fragments produced during the activation process promote the secretion of cytokine causing inflammation.

Previous reports described expression of MBL gene in a variety of cells such as CHO cells, HLF hepatoma cells or HEK293 EBNA cells. In particular, the expression level in CHO cells was very high but the produced MBL was mainly monomers and dimmers (Katsuki Ohtani et al., J. Immunol. Methods, 222: 135-144, 1999). The production of oligomeric form of MBL in HEK293 EBNA cells has been accomplished, but the gross productivity was less than 1 µg/mℓ (T. Vorup-Jensen et al., International Immunopharmacology, 1: 677-687, 2001). Production of oligomeric form of MBL is critically important, for the monomers and dimmers consisting of 3 subunits and 6 subunits, respectively, have little, if any, activities. It is also an important matter in developing a pharmaceutical product from MBL to develop a high producer cell line, since it is required in many milligram quantities.

MBL may also play an important role in adaptive immunity. Cells involved in phagocytosis such as neutrophils and macrophages play an important role in eliminating invading foreign microorganisms by phagocytosis. In addition to this, macrophage is an antigen presenting cell (APC), which activates T-cells, resulting in induction of acquired immune response. Therefore, multimeric MBL involved in activating complement system and as opsonin plays an important role not only in the primary defense as a component of innate immune system but also in the induction of acquired immune responses.

The amount of MBL in human serum varies according to individuals, ranging from 50 ng/mℓ to several µg/mℓ due to genetic variation of MBL gene. For example, when a point mutation occurred at codon 52; 54, or 57 of exon 1 of MBL gene, MBL molecules do not form oligomeric forms, producing non-functional smaller complexes consisting of not more than 3 MBL molecules. When mutation occurs in the promoter regions of MBL gene, the expression level of MBL was decreased, resulting in tremendous variation, of blood level MBL among affected individuals. In general, those infants or patients with neutropenia who have below certain level of MBL have weak immunity, becoming highly susseptible to microbial infections (Sumiya, M. et al., Lancet, 337: 1569-1570, 1991; Summerfield, J. A. et al., Lancet, 345: 886, 1995; Garred, P. et al., Lancet, 346: 941, 1995; Summerfield, J. et al., Br. Med. J., 314: 1229, 1997; Mullighan, C. G. et al., Scand. J. Immunol., 51: 111-122, 2000; Neth, O. et al., Lancet, 358: 614-618, 2001; Peterslund, N. A. et al., Lancet, 358: 637-638, 2001; Mullighan C. G et al., Blood, 99: 3 524-3529, 2002).

According to a study on hepatitis B virus chronic patients, the level of MBL in blood is a critical factor in the prognosis of the disease (Hakozaki Y et al., Liver 22, 29-34, 2002). Particularly, among patients developed fulminant hepatic failure from hepatitis B virus chronic infection, mortality of those who had over 3 µg/mℓ of blood level MBL was 0%, whereas those who had 1.5 µg/mℓ and under 0.5 µg/mℓ of blood level MBL it was 58% and over 80%, respectively. This study shows dramatic relationship between the blood level of MBL and mortality of patients with an infectious disease. This study also suggests that MBL supplement to the patients with low or no MBL may be beneficial.

In order for recombinant MBL to be used as a therapeutic agent, the recombinant MBL protein has to be produced in massive quantity for a reasonable price in the form of active oligomeric molecules. Unlike cytokines it is required in many milligrams per patients in each application. For example, human blood volume is average about 5 liters, requiring 25 mg to make 5 µg/ml MBL. Taking account of other body fluid volume, it could require much more in each application. Therefore it had been a critically important issue in the commercial product development out of MBL that one establishes not only a high producer recombinant cell line but also the cell line that produces active forms of polymeric MBL. Hocker B.A. et al. (2001) Faseb Journal, vol.15, no. 5, page A1012 describe the expression and characterization of human recombinant MBL retaining its higher-order oligomeric structure.

Thus, the present inventors made every effort to establish a recombinant cell line that could produce MBL in a commercial quantity and at the same time that could produce functional oligomeric form of MBL, which is capable of activating complement system by binding specifically to MBL binding glycoproteins in the presence of MASPs. As the result of this effort, the present inventors had completed this invention by confirming that active rhMBL suitable for therapeutic use could be produced in massive quantity in the form of multimeric polymer in a CHO. cell line transfected with a cloning vector containing a polynucleotide coding sequence for rhMBL.

### Description of Drawings

FIG 1 is a schematic diagram showing the genetic map of pMSG-MBL plasmid vector.
FIG 2A and FIG 2B are the pictures of MBL produced from the recombinant CHO cell line, CHO MBL/D1-3, that is transformed with pMSG-MBL. Fig. 2A is the SDS-PAGE analysis pattern, and 2B shows Western Blot analysis pattern using an MBL specific monoclonal antibody.
FIG 3 is a photograph of electron microscope showing oligomeric forms of MBL. Oligomeric MBL molecules consisting of 2,3,4,5,and 6 trimeric subunits of MBL are clearly seen.
FIG 4 is a photograph of electron microscope showing the complex of recombinant MBL protein and gold nanoparticle coated with pre S in the presence of calcium ion.
FIG. 5A and FIG 5B show that rhMBL binds specifically to pre-S, a glycoprotein, or mannan in quantitatively similar activity to the natural MBL.
FIG 6 shows the relative binding capacity of larger polymer form of MBL and smaller form consisting mainly of monomers and dimmers of trimeric subunits. Smaller forms have little, if any, binding capacity.
FIG 7 shows the comparison of the C4 activating capacities of larger form and smaller form of rhMBL.
FIG 8 shows that the rhMBL specifically binds to various microorganisms.
FIG. 9 shows that rhMBL inhibits infection of SARS-CoV into embryonic monkey kidney cells (FRhK-4).
FIG 10 is a set of phase constrast microscopic images showing FRhk-4 cells infected with SARS-CoV Without rhMBL treatment, cells are looking not healthy, whereas with MBL healthy cells are present.

### Disclosure

### Object of the Invention

It is the object of the present invention to construct a CHO cell line that is transfected with a vector containing a polynucleotide coding sequence for human MBL for the purpose of producing functional oligomeric form of MBL in massive quantity to be used for commercial purpose.

Further it is the object of present invention to establish a method for purification of rhMBL from the culture media of the recombinant CHO cell line.

### Summary of the Invention

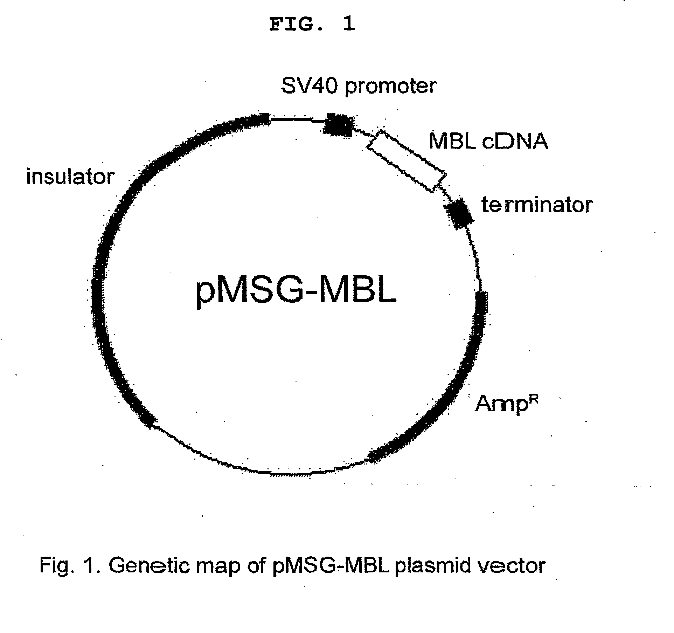

The present invention provides a CHO host cell transfected with an expression vector pMSG-MBL containing the sequence of rhMBL coding region represented in the plasmid map of FIG 1.

The present invention further provides a method for the mass-production of multimeric recombinant human MBL according to the following steps:
(1) preparing CHO host cells transfected with an expression vector pMSG-MBL containing the sequence of human MBL coding region;
(2) producing a recombinant human MBL from the transformed cell line with the expression vector with the sequence of human MBL coding region in a culture system; and
(3) purifying the recombinant human MBL prepared in the step (2).

### Detailed Description of the Invention

The present invention provides a CHO host cell transfected with an expression vector pMSG-MBL containing a polynucleotide coding for rhMBL represented in the plasmid map of FIG 1, which is sufficient enough to express olygomeric recombinant human MBL that can activate complement system by combining specifically with a MBL binding glycoprotein in the presence of MBL associated serine proteases.

Human MBL cDNA (Gene Bank NM_000242) was inserted in pMSG vector (Accession No: KCCM(Korean culture center of microorganisms) 10202) containing MAR element (nuclear matrix attachment region element) of beta-globin gene, poly-A of SV40 virus and transcription terminator complex of gastrin gene, resulting in the construction of a unique vector named pMSG-MBL.

The present invention thus provides host cells transfected with the expression vector containing the polynucleotide coding for rhMBL which is sufficient enough to express the recombinant human MBL, which is capable of activating complement system by binding specifically to a glycoprotein on the surface of microorganisms in the presence of MBL associated serine proteases.

Host cells used in the present invention are CHO (Chinese hamster ovary) cells,

In a preferred embodiment of the present invention, a transformant was prepared by introducing a cloning vector pMSG-MBL containing the polynucleotide encoding rhMBL, which is sufficient enough to express a recombinant MBL capable of activating complement system by binding specifically to glycoproteins on the surface of microorganisms in the presence of a MBL associated serine proteases, into a host cell line such as CHO cell, and then adapting the transformants to the condition with MTX (methotrexate), leading to the selection of the transformant which was able to mass-express rhMBL protein in the form of multimeric polymer. The selected transformant was named as MBL D1-3 (CHO cell line) and deposited at Korean Collection for Type Culture, Daejeon, Korea, on May 16, 2003 (Accession No: KCTC 10472BP).

The recombinant human MBL produced from the transformant described in the present invention is mainly in the form of multimeric forms, which is similar to the natural MBL from human blood. The cell line established in the present invention produced mainly functional multimeric form of MBL and showed high expression level, making it possible to use the transformed CHO cell line for mass-production of functional rhMBL protein.

The present invention further provides a method for the production of a recombinant human MBL, comprising the following steps:
(1) Method providing host cells transfected with a DNA construct including a cloning vector containing the polynucleotide encoding human MBL, which is sufficient enough to express a recombinant human MBL capable of activating complement system by binding specifically to a glycoprotein on the surface of microorganisms in the presence of MBL associated serine protease.
(2) Method producing recombinant human MBL by expressing the polynucleotide encoding human MBL in animal cell and from the cell employing a massive cell culture system; and
(3) Method purifying the recombinant MBL produced in the step (2). Particularly, exploiting the characters of MBL binding to glycoprotein of the step (1) and the MBL binding glycoprotein is exemplified by glycosylated viral envelope protein containing pre-S of HBV, glycosylated bacterial protein, glycosylated fungal protein, and synthetic glycoprotein.

In a preferred embodiment of the present invention, the producing step of a recombinant MBL of step (2) is characterized by the steps of suspension-culturing the cells in a serum-free/protein-free medium and sub-culturing those cells adapted well to the serum-free/protein-free medium to scale up gradually, resulting in the mass-production of rhMBL protein in the form of multimeric polymer.

In the step (3), a recombinant MBL protein was purified from the culture media of MBL gene transformants by using anion exchange chromatography and MBL binding capacity to pre-S of hepatitis B virus. The purification method of MBL is composed of the following steps: (a) fractionating samples containing multimeric recombinant human MBL by using anion exchange chromatography; (b) preparing column by packing substrate to which hepatitis B virus pre-S was immobilized; (c) capturing the recombinant MBLs specifically on to the immobilized hepatitis B virus pre-S by passing through the column a sample containing the recombinant MBL proteins in the presence of calcium ion, after equilibrating the column with a calcium ion containing buffer; and (d) eluting the recombinant MBL proteins by adding a buffer solution supplemented with EDTA or EGTA to the column onto which the recombinant MBL was captured in the step (c).

In the step (a), a material which was generally used for anion exchange chromatography was used, and Q-sepharose was one of such column material used. The sample containing the MBL proteins in the form of monomer or dimer was fractionated in the presence of 150 - 200 mM NaCl, and the other portion containing a high molecular weight MBL protein in the form of copolymer was eluted in the presence of 350 - 400 mM NaCl.

In the step (b), a substrate that was generally used for affinity chromatography was used, and sepharose was one of the general substrates.

In the step (c), equilibrium of column was performed by adding a buffer solution or the same solution as used for the affinity binding of a recombinant MBL and hepatitis B virus pre-S. The binding of recombinant MBL to hepatitis B virus pre-S was preferably performed in the presence of calcium ion, and at that time, the concentration of calcium was preferably 2 - 20 mM. A sample containing a recombinant MBL protein could be supernatant obtained from MBL producing cell transformant culture medium or MBL solution eluted from MBL fractionation column mentioned above.

In the step (d), the elution of rhMBL protein was performed using EDTA or EGTA buffer solution in the absence of calcium ion. The buffer solution could be the solution containing EDTA or EGTA at the concentration of 2 - 10 mM, for example, distilled water or Tris-Cl buffer, pH 7.4.

The eluent prepared in the step (d) was freeze-dried or dialyzed before freeze-drying, resulting in a purified recombinant MBL protein.

The purification method of rhMBL protein of the present invention is also applicable for purifying MBL obtained from natural biological samples, in addition to recombinant MBL protein prepared from the transformant. The biological sample can be exemplified by blood, plasma or serum.

The recombinant human MBL of the present invention is similar to the natural form purified from human blood. It is oligomeric forms and active in binding to microorganism thereby activating complement system. And high yield production level (50 µg/million cells/day) ensures commercial production of functional MBL for product development. So produced rhMBL may be effectively used for the treatment of an individual infected with virus, bacteria or fungi. It can also be used for the production of a diagnostic kit for detecting the recombinant human MBL.

### Examples

Practical and presently preferred embodiments of the present invention are illustrative as shown in the following Examples.

### Example 1: Preparation of a MBL gene transformant and expression of a recombinant human MBL

### <1-1> Construction of an expression vector

pEZ-MBL 2-5 was constructed by cloning MBL cDNA obtained by PCR from human hepatocyte cDNA library into pEZ vector, and then the nucleotide sequence of MBL cDNA was confirmed to be authentic from the known nucleotide sequence of MBL (Gene Bank NM_000242). PCR was performed by using the pEZ-MBL 2-5 as a template and using a primer set (forward primer 1 and backward primer 2) to amplify about 750 kb sized MBL cDNA. Each primer contains restriction enzyme recognition site and Kozak sequence, so the whole coding region was amplified. Then, pMSG-MBL was prepared by cloning MBL cDNA into pMSG vector (Accession No: KCCM 10202), and then the inserted nucleotide sequence of MBL was confirmed (FIG 1).
Forward primer 1 (SEQ. ID. No 1)
   ctagctagcc accatgtccc tgtttccatc actc (34mer)
Backward primer 2 (SEQ. ID. No 2)
   gaagatctca gatagggaac tcacagacg (29mer)

### <1-2> Transformation of host cell with pMSG-MBL

### <1-2-1> Preparation of pMSG-MBL expression plasmid DNA

*E. coli* was transfected with pMSG-MBL, and the resultant transformant was cultured in 100 mℓ of LB medium supplemented with 100 µg/mℓ of ampicillin (Sigma, USA). The pMSG-MBL DNA was separated by plasmid purification kit (QUIAPREP Plasmid Midi Kit, Quiagen, USA). The purified DNA was digested with restriction enzyme *Sca* I to make it linear, which was separated by PCR product purification kit (QIAQuick PCR product purification kit, Quiagen, USA).

### <1-2-2> Preparation of host cells

After culturing CHO DG44 (dhfr-/dhfr-) host cells in α-MEM medium supplemented with 10% cFBS, the number of cells was counted by hemacytometer. The cells were distributed (2 x 10⁵cells/well) in α-MEM medium supplemented with 10% cFBS, which were cultured in a CO₂ incubator for 24 hours.

### <1-2-3> Transformation

2 µg of pMSG-MBL vector was mixed with 5.3 µℓ of Dosper™ and 16 ng of pDCHIP vector (plasmid containing DHFR gene, Venolia, L. et al., 1987, Somat. Cell Mol. Genet. 13, 491-501) DNA to induce reaction at room temperature for 45 minutes. The resultant product was added to host cells. The cells were cultured at 37°C for 6 hours, then medium was replaced with fresh α -MEM medium containing 10% cFBS (3 mℓ/well), followed by further culture. 2-3 days later, when transformed cells were grown enough, trypsin was added and 2 mℓ of α -MEM (w/o) containing 10% dFBS was added to the cells (4 x 10⁵ cells/well), followed by further culture again. Every 2-3 days, medium was replaced with a fresh one, and the condition of cells and the formation of single colony were observed under microscope. 10 days later, adapted early cells were obtained.

### <1-3> Selection of a MBL expressing cell line and amplification of the MBL gene

After obtaining adapted early cells, the MTX (methotrexate) concentration in medium was increased gradually to induce amplification of MBL gene inserted in the transformed cells. The cells were distributed by 4 x 10⁵ cells/well, and then cultured in a medium (α-MEM + 10% dFBS) supplemented with 10 nM MTX until confluence, during which the medium was replaced with a fresh one every 2-3 days. The cells were distributed again by 4 x 10⁵ cells/well, and adapted to a medium supplemented with 100 nM MTX by following the same procedure as used above, and then adapted again to 1 uM MTX condition. During the amplification of the MBL gene, western blot analysis was performed in every stage to observe the changes of expression level of MBL. In this the expression of MBL was increased with the increase of MTX content in medium.

### <1-4> Selection of a single cell line

In order to separate a single cell line showing high MBL expression rate, cells cultured in a medium (α-MEM + 10% dFBS) containing 1 uM MTX were distributed to a 9 6-well plate to make 0.5 cells/well, then cultured in a medium containing 1 uM MTX. After 2 weeks, when the formation of a single colony was confirmed, the cells were transferred to a 24-well plate, which was further cultured until cells were proliferated enough. Some of them were frozen and stored, and some of them were used for the comparison of expressions by Western blot analysis. And, a single cell D1-3 transformant, which was proved to produce high molecular weight form of MBL in a large quantity, was selected as a cell line of the present invention. The selected transformant was named as MBL D1-3 (CHO cell line) and deposited at Korean Collection for Type Culture, Daejeon, Korea, on May 16, 2003 (Accession No: KCTC 10472BP). PAGE analysis of the recombinant MBL protein expressed from the transformant was performed under non-denaturing condition and found to be multimeric form of MBL, which is very similar to the characteristics of human originated natural MBL.

### <1-5>Quantification of recombinant MBL expressed in a transformant

Based on the standard amount of purified MBL obtained from human serum, the expression level of recombinant MBL protein from transformed CHO MBL/D1-3 cells was estimated. Cells were distributed into T25 flask at a concentration of 5 x 10⁵, and then cultured in a medium (α-MEM(w/o) + 10% dFBS) until 90% confluence. Then, 3 mℓ of medium (α-MEM(w/o) + 5% dFBS) was added thereto and further cultured for 4 days. The culture medium was diluted 10 times, followed by Western blot analysis. The result was compared with the standard amount of natural MBL. From this the expression level of recombinant MBL protein was confirmed to be about 50 µg/10⁶ cells/day, as cultured on a cell culture flask.

### Example 2: Method for mass-production of rhMBL protein

### <2-1> Preparation of a cell line adapted for suspension culture in a serum-free medium

Anchorage-dependent MBL expressing cell line was cultured in α-MEM (w/o) medium supplemented with 10% dFBS and 1 uM MTX. After recovering viable cells, they were inoculated into a 250 ml spinner flask containing 100 ml of protein-free HyQ SFM4 CHO medium (Hyclone, USA) supplemented with 0.15% sodium bicarbonate at the inoculum size of 5 x 10⁵ cells/ml. The culture in the spinner flask was performed at 37°C in a 5% CO₂ incubator, which was stirred at 40 rpm whole time. When the number of cells reached 1.0 - 2.0 x 10⁶ cells/ml, cells were inoculated again into 100 ml HyQ SFM4 CHO medium supplemented with 0.15% sodium bicarbonate with the inoculum size of 5 x 10⁵ cells/ml. Viability of the cells was determined by trypan blue exclusion method. In particular, over 90% viability was confirmed in the late adaptation period.

The cells adapted to serum-free/protein-free medium were cultured until the cell density reached 2.5 x 10⁶ cells/mℓ. Then, the cells were recovered and re-suspended in a freezing medium. The cells were distributed into cryovials to make the concentration of 2.8 x 10⁷ cells/mℓ, which was then stored at liquid nitrogen tank.

### <2-2> Development of bioreactor culture by using a cell line adapted for serum-free suspension culture

Cells for suspension culture were inoculated to a 250 mℓ spinner flask containing 100 mℓ of serum-free/protein-free medium (HyQ SFM4 CHO medium) supplemented with 0.15% sodium bicarbonate with the inoculum size of 5 x 10⁵ cells/mℓ. When the cell number was increased to 1.0 - 2.0 x 10⁶ cells/mℓ, the cells were inoculated to a 500 mℓ spinner flask containing 200 mℓ of HyQ SFM4 CHO medium with the inoculum size of 5 x 10⁵ cells/mℓ. Then, when the cell number was increased again to 2 x 10⁶ cells/mℓ, the cells were inoculated to a 1,000 mℓ spinner flask containing 400 ml HyQ SFM4 CHO medium with the inoculum size of 5 x 10⁵ cells/mℓ. This way the cells were cultured scaling up gradually, resulting in the preparation of 1 L of seed culture with the concentration of 2.5 x 10⁶ cell/mℓ

The seed cell culture prepared above were inoculated to a 7.5 L (5 L working volume) bioreactor and cultured at 34 °C for 5 days (50 rpm, DO 50, pH 7.2 - 7.4).

### Example 3: Method for the purification of multimeric recombinant human MBL protein

MBL expressing CHO cell was cultured in HyQ SFM4 CHO medium, and then the culture medium was centrifuged and passed through a 0.45 µm membrane filter to remove cells. Anion exchange chromatography and affinity chromatography were performed to purify recombinant MBL proteins. The quantity of purified proteins was determined by using MBL ELISA kit.

### <3-1> Fractionation of sample containing multimeric polymer by using Q-sepharose column

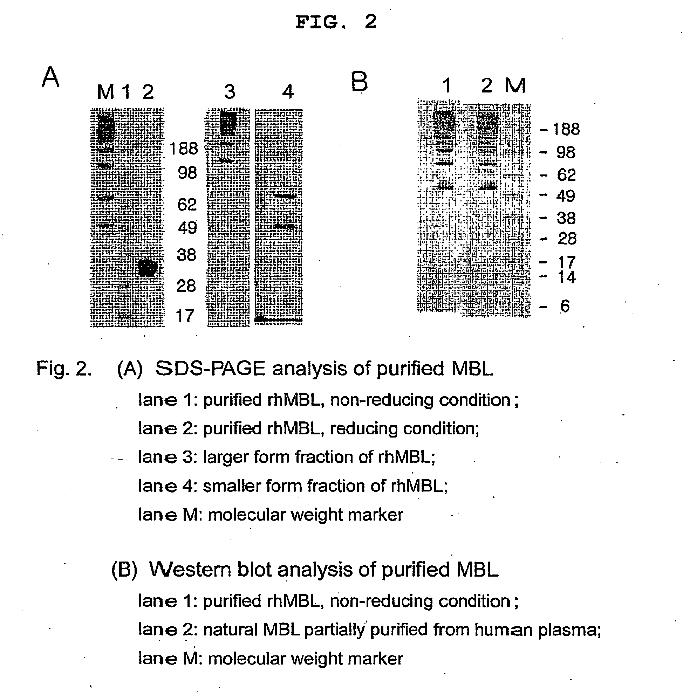

After optimizing pH and conductivity of supernatant, smaller form of MBL such as monomer and dimer was removed by using a column packed with Q-sepharose, and only MBL in larger forms was collected. The sample containing smaller form of MBL was fractionated by passing through 150 - 200 mM NaCl buffer solution, and the sample containing larger form of MBL was eluted with 350 - 400 mM NaCl buffer solution. This result showed that more than 80% were expressed in high molecular copolymer form of MBL from the cell line that is established in this invention (FIG 2A).

### <3-2> Preparation of pre-S-sepharose column

Recombinant pre-S protein used in the present invention was obtained by expressing pre-S gene, which is a portion of surface protein of hepatitis B virus, in *Sacharomyces cerevisiae,* and then purifying it in the form of highly glycosylated molecules (International Patent Publication No. WO 02/094866).

One gram of sepharose 4B powder activated by CNBr was dissolved in 1 mM HCl, followed by washing several times. In order to use recombinant pre-S protein as a ligand, 6.4 mg of pre-S protein was dissolved in coupling buffer (0.2 M NaHCO₃, 0.5 M NaCl and pH 8.3), making the concentration of 0.5 - 10 mg/mℓ CNBr activated Sepharose 4B resins were mixed with the pre-S solution and reacted at room temperature for 2, hours. Then, the mixture was transferred to blocking buffer (0.1 M Tris-Cl, pH 8.0), followed by further reaction at room temperature for 2 hours. After washing, pre-S immobilized to sepharose was confirmed by western blot analysis and found to be almost all pre-S recombinant proteins were immobilized to the sepharose column, which was used for the purification of a recombinant MBL protein. Finally, pre-S-sepharose column for the purification of recombinant human MBL was prepared by packing pre-S-sepharose 4B resin in a column.

### <3-3> Purification of a recombinant human MBL

The column filled with pre-S-sepharose was equilibrated with a binding buffer (20 mM Tris, 150 mM NaCl and 10MM CaCl₂, pH 7.6). The column fraction from the Q-Sepharose containing recombinant MBL proteins or a culture media containing MBL was loaded onto the column to adsorb MBL. Then, the column was washed several, times with the binding buffer. The eluting buffer (20 mM Tris, 150 mM NaCl and 5mM EDTA, pH 7.6) without Ca²⁺ was passed through the column to elute recombinant human MBL. The obtained eluent was investigated with SDS-PAGE. As a result, purified recombinant human MBL showing 99.9% purity was obtained (FIG 2A and FIG 2B).

### Example 4: Verification of a multimeric recombinant human MBL by EM and by MBL binding activity to the surface of a microorganism

### <4-1> Confirmation of the formation of a multimeric recombinant human MBL

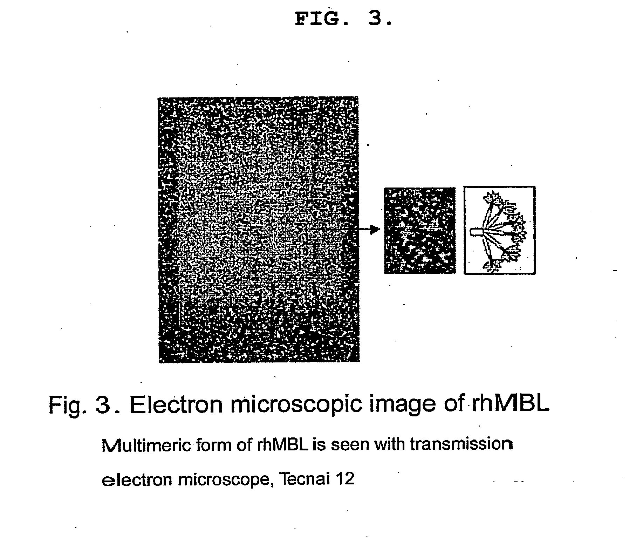

In order to confirm whether or not a purified recombinant human MBL formed a multimeric polymer in the shape of a flower bouquet, the purified recombinant human MBL was created with amorphous carbon, and then the shape of the protein was observed under transmission electron microscope (Tecnai 12, FEI, Netherlands) and found the purified recombinant human MBL to be multimeric polymers in the shape of a bouquet (FIG 3).

### <4-2> Binding of recombinant human MBLs to pre-S coated gold particles

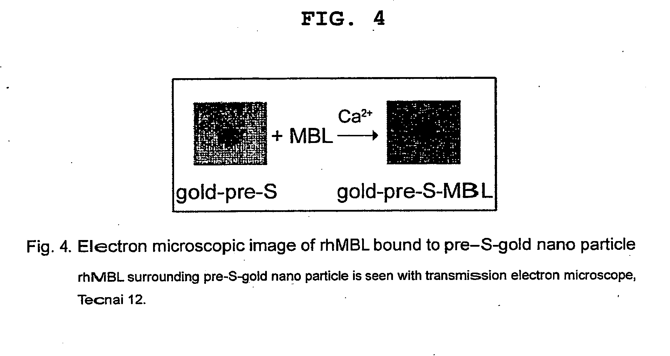

The binding capacity of recombinant human MBL to the surface of a microorganism was observed by using pre-S, a surface protein of hepatitis B virus. Pre-S proteins were attached to the surface of gold particles which is of 20 - 40 nm size, which was, thus, made as if a microorganism. Recombinant human MBLs were added thereto in the presence of calcium. Then, whether recombinant human MBL was attached to the pre-S proteins was observed under transmission electron microscope Tecnai 12, resulting in the confirmation of a complex formation. As shown in FIG 4, pre-S proteins coated on the gold particles were surrounded by recombinant human MBLs in the presence of calcium ion. This demonstrates how rhMBL binding to the surface of a microorganism looks like. In actual case, MBL binding to microorganism might mechanically block infectivity of the organism-specially, such as viruses-to the cells, in addition to activating complement system and acting as an opsonin.

### Example 5: Confirmation of binding activity of recombinant human MBL

In order to investigate the biological activity of rhMBL, binding activity to pre-S and mannan was assayed in the presence of calcium ion.

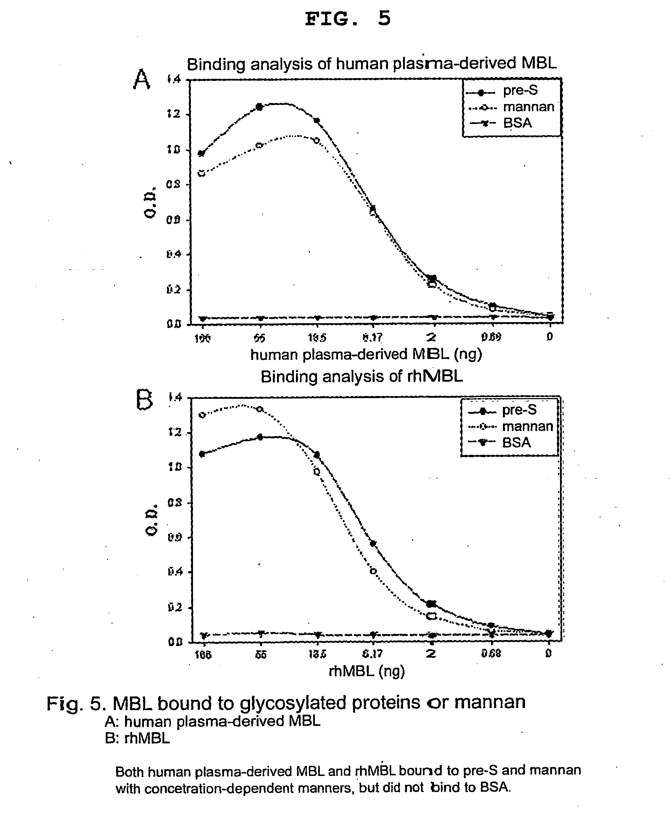

Mannan or pre-S protein of hepatitis B virus dissolved in 50 mM carbonate-bicarbonate buffer solution was distributed into a microtiter plate (Nunc Maxisorp Immunoplate) to make 1 µg per well; which was then incubated for overnight at 4°C . The pre S or mannan coated plate was washed with a washing buffer (20 mM Tris, 150 mM NaCl, 10 mM CaCl₂, 0.05% Tween-20, pH 7.6) four times, followed by blocking with 0.2% BSA at room temperature for one hour. After washing with the washing buffer three times, enough MB L was added to each well to make the MBL quantity in each well as indicated in the figure. To do this 1 µg of recombinant human MBL in 1ml of binding buffer (20 mM Tris, 1 M NaCl, 10 mM CaCl₂, 0.1% BSA, 0.05% Tween-20, pH 7.6) were made serial dilution to make the necessary amount of MBL in 100 µℓ, which was then added onto each well and incubated at room temperature for 2 hours. The plates were washed with the washing buffer 6 times again. Then, mouse monoclonal anti-human MBL antibodies (MBL8F6, Dobeel Corp., Korea) were diluted 10,000 fold and added 100 µℓ onto each well followed by incubating at 37 degree C for 1 hour. Anti-mouse IgG-HRP was diluted 10,000 fold and added 100ul to each well. The plate was incubated at 37 degree C for one hour and then, the color was developed by adding 150 µℓ of TMB solution. After 20 minutes, the reaction was terminated by the addition of 50 µℓ of 2 M H₂SO₄- OD₄₅₀ was measured using ELISA plate reader (Multiskan Ex, Labsystems, USA). FIG 5A shows the binding of natural MBL purified from human serum to mannan and pre-S protein, and FIG 5B shows the binding of recombinant human MBL to the pre-S and mannan. In conclusion, recombinant human MBL and the natural MBL for human had similar binding activity to pre-S and mannan, but both of them did not bind to Bovine Serum Albumin (FIG 5A and FIG 5B).

### Example 6: Comparison of biological activity between high molecular weight multimeric recombinant human MBL and low molecular weight oligomeric recombinant human MBL

The biological activity of the larger and smaller form of recombinant human MBL was investigated in order to confirm which one of recombinant human MBL proteins, larger form or smaller form including monomer and dimer; binds more efficiently to pre-S and to mannan in the presence of calcium ion.

### <6-1> MBL binding to glycosylated protein pre-S

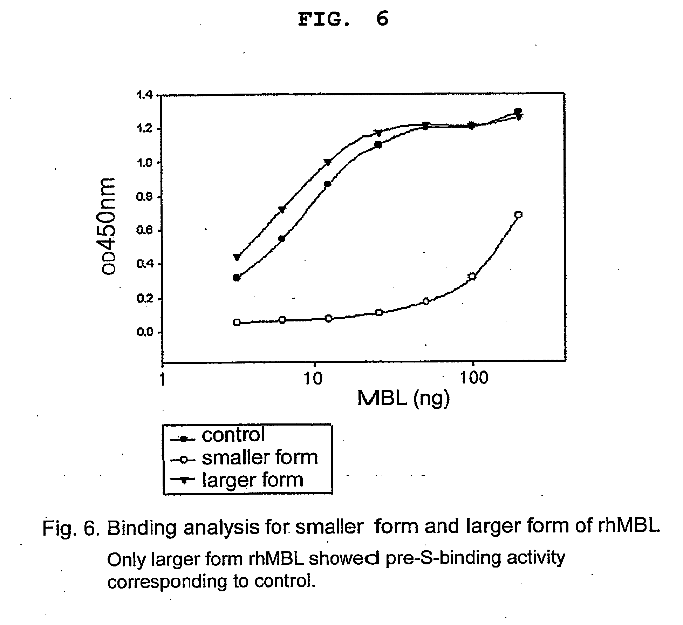

Pre-S proteins were coated on a plate (Nunc Maxisorp Immunoplate) under the same condition as described in the Example 5. Then, the larger form or smaller form was dissolved in a binding buffer (20 mM Tris, 1 M NaCl, 10 mM CaCl₂, 0.1% BSA 0.05% Tween-20, pH 7.6) and added onto pre-S coated plate at the different concentrations to make desired amount in each well. And the rest of the binding assays were performed as in the example 5. As described earlier, MBL binding activity to pre-S proteins was much greater with multimeric recombinant human MBL compare to the smaller form containing mainly monomers and dimers (FIG 6).

### <6-2> Activation of C4 in the complement system

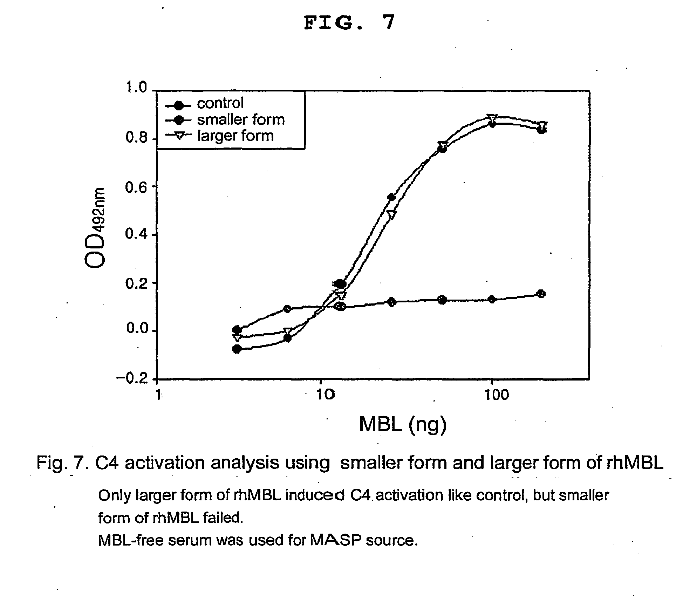

Nunc Maxisorp Immunoplate was coated with 500 ng of pre-S proteins per well, to which different concentrations of recombinant human MBLs were added, allowing MBL binding to pre-S at room temperature for 2 hours. As a source of MASP protein, 100 ul of MBL-free serum diluted 100 fold was added to each well and incubated 90 minutes at room temperature. The plate was washed with a washing buffer 6 times, then 500 ng of C4 was added, followed by further reaction at room temperature for 2 hours. Anti-C4 antibody-HRPs were diluted 2000 fold and 100 ul was added to each well. Then, allowed the reaction to take place at room temperature for one hour. One hundred fifty µℓ of OPD solution was added, and color was development for 20 minutes. Then, C4b deposit was measured after adding 50 µℓ of 3 M HCl and reading the OD at OD₄₉₂ using an ELISA plate reader (FIG 7). For the experiments, both multimeric recombinant human MBLs in larger form provided by the present invention and recombinant human MBLs in smaller form such as monomers or dimers were used respectively for the comparison of their activities. As shown in FIG 7, C4 was greatly activated by multimeric recombinant human MBL in larger form but was hardly activated by those proteins in smaller form. Therefore, it was confirmed that recombinant human MBLs in the form of multimeric polymer provided by the present invention have an excellent activity for the C4 activation.

### Example 7: Binding of recombinant human MBL to various microorganisms

Eleven strains including both Gram-positive bacteria and Gram-negative bacteria and fungi were cultured in liquid medium appropriate for each of them. To immobilize the cultured bacterial cell, 100 ul of cultured cells were distributed in a plate (Maxisorp Immunoplate, Nunc) to make 1 x 10⁶ cells/well and incubated at 4°C for overnight. Blocking was performed by using 0.2% BSA at 37 °C for one hour. Five hundred ng of recombinant human MBL dissolved in 100ul binding buffer (20 mM Tris, 1 M NaCl, 10 mM CaCl₂, 0.1 % BSA, 0.05% Tween-20, pH 7.6) was added to each well, followed by reaction at 37°C for one hour. Anti-human MBL mouse monoclonal antibody MB1B5 (Dobeel Corp., Korea) was diluted 10,000 fold and added 100 µℓ to each well, followed by reaction at 37°C for one hour. Anti-mouse IgG-HRP was diluted 10,000 fold and added 100ul to each well, followed by reaction at 37°C for one hour. Then, 100 *µℓ* of TMB solution was added thereto and color was development for 30 minutes. The reaction was terminated by adding 50 µℓ of stop solution (H₂SO₄), and then OD₄₅₀ was measured by using ELISA plate reader (Multiskan Ex, Labsystems, USA).

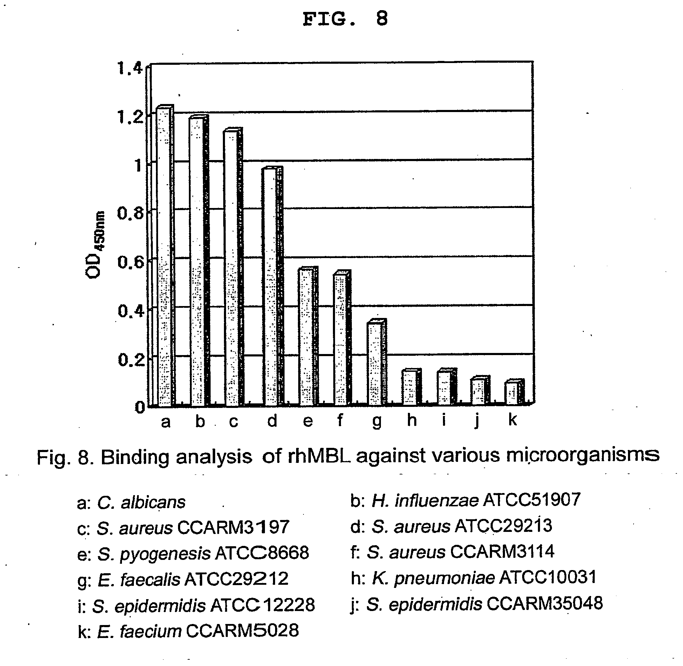

The microorganisms used in the present invention showed different binding activity to a recombinant human MBL, and as shown in FIG 8, C. *albicans, H. influenzae* ATCC 51907, S. *aureus* CCARM 3197 and S *aureus* ATCC 29213 showed very high binding activity to a recombinant MBL protein, but S *pyrogenes* ATCC 8668, *S aureus* CCARM 3114 and *E. faecalis* ATCC 29212 showed a medium level of binding activity. *K pneumoniae* ATCC 10031, S. *epidermis* ATCC 12228, *S. epidermis* CCARM 35048 and *E. faecium* CCARM 5028 had low binding activity to MBL. In conclusion, it was proved that a recombinant human MBL, like a natural MBL, binds to microorganisms, recognizing the pattern of a glycosylated surface protein and binding activity varies with target microorganisms.

### Example 8: Inhibition of SARS-Co V infection by recombinant human MBL

FRhk-4 cells, monkey kidney cells, were cultured in MEM. To the culture solution, added was recombinant human MBL, which was then infected with SARS-CoV The recombinant human MBL was diluted with cell culture media in four-fold serially starting from concentration of 2.5 µg/mℓ, and SARS-CoV, primary isolate from patient, was used (Ksiazek TG et al. N. Eng. J. Med 348, 1953-1966, 2003; Peiris et al., Lancet 361,1319-1325, 2003).

In order to confirm whether or not the host cells were infected with SARS-CoV and replicated, quantitative real-time PCR (GeneAmp PCR system, Applied Biosystems, USA) was performed with a set of SARS-CoV specific primers. FRhk-4 cells infected with SARS-CoV and not treated with a recombinant human MBL were used as a control group.

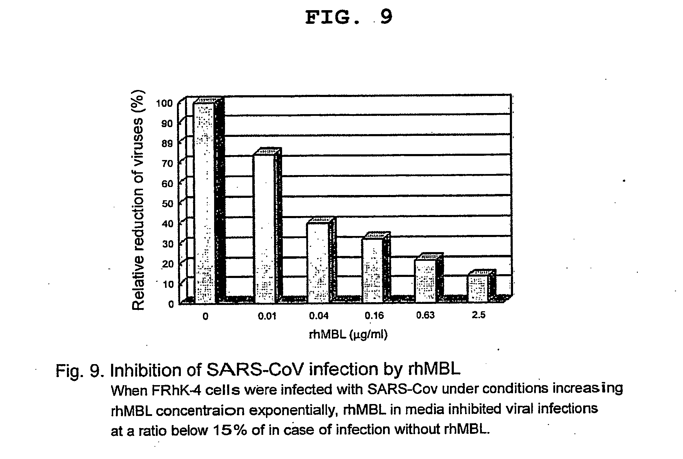

As shown in FIG 9, the infection with SARS-CoV was inhibited by the treatment of recombinant human MBL in dose-dependent manner. For example, when cells were cultured in the presence of Recombinant human MBL by the concentration of 2.5 µg/mℓ, the proliferation of the virus was inhibited greatly (less than 15% proliferation), comparing to a control group. This 15% is most likely from the virus inoculum.

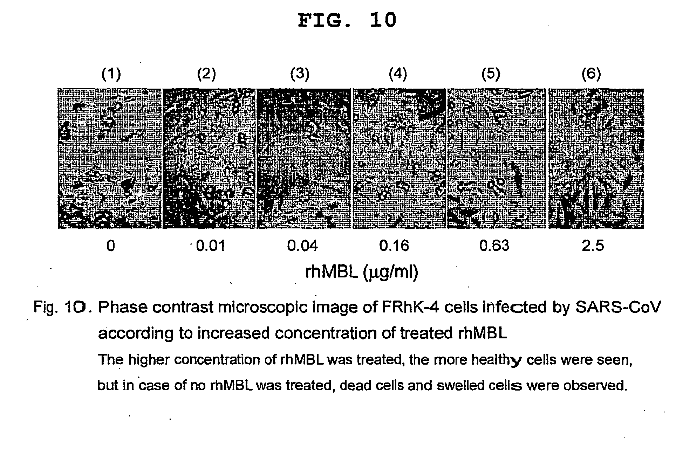

FIG 10 is a photograph of phase contrast microscope showing FRhk-4 cells infected with SARS-CoV In FIG 10 (1), healthy cells were not observed in a control group that was not treated with recombinant human MBL, but in FIG 10 (2) - (6), healthy cells were observed in testing groups treated with recombinant human MBL. In particular, the number of healthy cells was increased with the increase of the concentration of recombinant human MBL.

### Industrial Applicability

As exemplified hereinbefore, methods for production of multimeric Recombinant human mannose binding lectin (rhMBL) in massive quantity presented in this invention is a significant advancement in the development of VIBL as a useful commercial product. In particular multimeric form of MBL efficiently binds to microorganisms, thereby it could efficiently inhibit infection of virus, bacteria, or fungi, so that it might be used for the development of a medicine for the prevention and/or the treatment of infection with microorganisms such as virus, bacteria, fungi, in particular SARS-CoV, and the production of diagnostic kit for the detection of human MBL.
## Claims

1. A CHO host cell line transfected with an expression vector pMSG-MBL illustrated in a plasmid map of FIG. 1 containing a polynucleotide coding for human mannose binding lectin (human MBL).

2. The CHO host cell line according to claim 1, wherein the cell line is the CHO cell line MBL D1-3 deposited at Korean Collection for Type Culture under accession No: KCTC 10472BP.

3. A method for the mass-production of multimeric recombinant human MBL (rhMBL) comprising the following steps:
(1) preparing CHO host cell lines transfected with an expression vector pMSG-MBL illustrated in a plasmid map of FIG. 1 containing the sequence of human MBL coding region;
(2) producing rhMBL by expressing the sequence of human MBL coding region in said CHO host cells and from the culture of that cells; and
(3) purifying rhMBL prepared in the step (2).

4. The method according to claim 3, wherein the CHO host cell line is the CHO host cell line MBL D1-3 deposited under accession No: KCTC 10472BP.

5. The method according to claim 3, wherein the step (2) is **characterized by** the following steps:
suspension-culturing the cells in a serum-free/protein-free medium; and
sub-culturing those cells adapted well to the serum-free/protein-free medium to scale them up gradually, resulting in the mass-production of rhMBL protein in the form of multimeric polymer.

6. The method according to claim 3, wherein the step (3) is **characterized by** the following steps:
(a) fractionating samples containing multimeric rhMBL by using anion exchange chromatography;
(b) preparing column by packing substrate to which hepatitis B virus pre-S was conjugated;
(c) binding the rhMBL to the hepatitis B virus pre-S specifically by adding a sample containing the rhMBL to the column in the presence of calcium ion, after equilibrating the column; and
(d) eluting the rhMBLs by adding a buffer solution supplemented with EDTA or EGTA from the column onto which the rhMBL was bound.

7. The method according to claim 3, wherein the rhMBL is capable of activating complement system by binding specifically to a glycoprotein on the surface of microorganisms in the presence of MBL associated serine protease, said glycoprotein being selected from a group consisting of pre-S glycosylated viral envelope protein, glycosylated bacterial protein, glycosylated fungal protein, and synthetic glycoprotein.

## Patentansprüche

1. . CHO-Wirtszelllinie, transfiziert mit einem in einer Plasmidkarte von Fig 1 dargestellten Expressionsvektor pMSG-MBL, der ein für humanes Mannose-bindendes Lektin (humanes MBL) kodierendes Polynukleotid enthält.

2. . CHO-Wirtszelllinie nach Anspruch 1, wobei die Zelllinie die CHO-Zelllinie MBL D1-3 ist, die bei der Korean Collection for Type Culture unter der Hinterlegungsnr KCTC 10472BP hinterlegt ist.

3. . Verfahren zur Massenherstellung von multimerem rekombinantem humanem MBL (rhMBL), umfassend die folgenden Schritte:
(1) Herstellen von CHO-Wirtszelllinien, die mit einem in einer Plasmidkarte von Fig 1 dargestellten Expressionsvektor pMSG-MBL transfiziert sind, der die Sequenz der für humanes MBL kodierenden Region enthält;
(2) Herstellen von rhMBL durch Exprimieren der Sequenz der für humanes MBL kodierenden Region in den CHO-Wirtszellen und von der Kultur dieser Zellen; und
(3) Reinigen des in dem Schritt (2) hergestellten rhMBL

4. Verfahren nach Anspruch 3, wobei die CHO-Wirtszelllinie die unter der Hinterlegungsnr KCTC 10472BP hinterlegte CHO-Wirtszelllinie MBL D1-3 ist

5. . Verfahren nach Anspruch 3, wobei der Schritt (2) durch die folgenden Schritte **gekennzeichnet** ist:
Suspensionskultivierung der Zellen in einem serumfreien/proteinfreien Medium; und
Subkultivierung dieser gut an das serumfreie/proteinfreie Medium angepassten Zellen, um sie schrittweise aufzuskalieren, woraus sich die Massenherstellung von rhMBL-Protein in Form eines multimeren Polymers ergibt

6. . Verfahren nach Anspruch 3, wobei der Schritt (3) durch die folgenden Schritte **gekennzeichnet** ist:
(a) Fraktionieren von multimeres rhMBL enthaltenden Proben unter Verwendung der Anionenaustauschchromatographie;
(b) Herstellen der Säule durch Bepacken mit Substrat, an welchem das Prä-S des Hepatitis-B-Virus konjugiert wurde;
(c) spezifisches Binden des rhMBL an das Prä-S des Hepatitis-B-Virus durch Zusatz einer das rhMBL enthaltenden Probe zu der Säule in Gegenwart von Calciumion nach Äquilibrieren der Säule; und
(d) Eluieren der rhMBLs durch Zusatz einer mit EDTA oder EGTA supplementierten Pufferlösung aus der Säule, an welche das rhMBL gebunden wurde

7. . Verfahren nach Anspruch 3, wobei das rhMBL in der Lage ist, das Komplementsystem durch spezifisches Binden an ein Glykoprotein an der Oberfläche von Mikroorganismen in Gegenwart von MBL-assoziierter Serinprotease zu aktivieren, wobei das Glykoprotein aus einer Gruppe bestehend aus Prä-S-glykosyliertem viralem Hüllprotein, glykosyliertem Bakterienprotein, glykosyliertem Pilzprotein und synthetischem Glykoprotein ausgewählt ist

## Revendications

1. Lignée cellulaire hôte CHO transfectée avec un vecteur d'expression pMSG-MBL illustré dans une carte de plasmide de la figure 1 contenant un polynucléotide codant pour la lectine de liaison du mannose humaine (MBL humaine).

2. Lignée cellulaire hôte CHO selon la revendication 1, dans laquelle la lignée cellulaire est la lignée cellulaire CHO MBL D1-3 déposée à la Collection Coréenne de Cultures Types sous le No d'ordre KCTC 10472BP

3. Procédé pour la production en masse de MBL humaine recombinante multimérique (rhMBL) comprenant les étapes suivantes
(1) la préparation de lignées cellulaires hôtes CHO transfectées avec un vecteur d'expression pMSG-MBL illustré dans une carte de plasmide de la figure 1 contenant la séquence d'une région codante de la MBL humaine ;
(2) la production de rhMBL par l'expression de la séquence de région codante de la MBL humaine dans lesdites cellules hôtes CHO et à partir de la culture de ces cellules ; et
(3) la purification de la rhMBL préparée à l'étape (2)

4. Procédé selon la revendication 3, dans lequel la lignée cellulaire hôte CHO est la lignée cellulaire hôte CHO MBL D1-3 déposée sous le No d'ordre : KCTC 10472BP

5. Procédé selon la revendication 3, dans lequel l'étape (2) est **caractérisée par** les étapes suivantes:
une culture en suspension des cellules dans un milieu sans sérum/sans protéine ; et
une sous-culture de ces cellules bien adaptées au milieu sans sérum/sans protéine afin de les étendre progressivement, résultant en la production en masse de protéine rhMBL sous la forme d'un polymère multimérique

6. Procédé selon la revendication 3, dans lequel l'étape (3) est **caractérisée par** les étapes suivantes :
(a) le fractionnement des échantillons contenant rhMBL multimérique par l'utilisation d'une chromatographie par échange d'anions ;
(b) la préparation d'une colonne par remplissage avec un substrat auquel le virus de l'hépatite B pre-S a été conjugué ;
(c) la liaison de la rhMBL au virus de l'hépatite B pre-S de manière spécifique par l'ajout d'un échantillon contenant la rhMBL à la colonne en présence d'ion calcium, après équilibration de la colonne ; et
(d) l'élution des rhMBL par l'ajout d'une solution tampon supplémentée avec de l'EDTA ou de l'EGTA de la colonne sur laquelle la rhMBL a été liée

7. Procédé selon la revendication 3, dans lequel la rhMBL est capable d'activer le système du complément en se liant spécifiquement à une glycoprotéine sur la surface des micro-organismes en présence de sérine protéase associée à la MBL, ladite glycoprotéine étant choisie dans un groupe constitué par une protéine d'enveloppe virale glycosylée de pre-S, une protéine bactérienne glycosylée, une protéine fongique glycosylée, et une glycoprotéine de synthèse
